# EUROPEAN PATENT SPECIFICATION

(11) **EP 1 676 544 B1**
(45) Date of publication and mention of the grant of the patent: **24.02.2010**
(21) Application number: 05257967.9
(22) Date of filing: 22.12.2005
(51) Int. Cl.: A61F 2/01

(54) **Distal protection filter with improved wall apposition**
Distaler Schutzfilter mit verbesserter Apposition zu den Wänden
Filtre de protection distal avec apposition au paroi ameliorée

(30) Priority: 30.12.2004 US 26309
(43) Date of publication of application: 05.07.2006
(73) Proprietor: Cordis Corporation, Miami Lakes, FL 33014 (US)
(72) Inventor: Park, Jin S., Parsippany, NJ 07054 (US); Wang, Huisun, Miramar, FL 33027 (US); Widenhouse, Christopher William, Cincinnati, OH 45249 (US)
(74) Representative: Gover, Richard Paul

(56) References cited:
- EP-A- 1 179 321
- EP-A- 1 386 624
- WO-A-01/45592
- WO-A-02/43595
- WO-A-02/060519
- US-A1- 2003 150 821
- US-A1- 2004 225 321
- US-B1- 6 652 554

## Description

The present invention relates to intravascular devices used to assist in medical treatment and procedures. More specifically, the present invention relates to a blood filtering system for preventing embolic material from migrating through a blood vessel during an intravascular procedure.

Atherosclerosis is a complex disease, being primarily a result of buildup in the arteries that may start as early as childhood and progress as one ages. Progression may be rapid in some people. Blood vessels may become completely occluded or more often narrowed and/or stenotic in a number of ways. A stenosis may be formed by an atheroma which is typically a harder, calcified substance which forms on the inside walls of the blood vessel. The stenosis may also be formed by a buildup of thrombus material, which may restrict blood flow through the vessel. In general, atherosclerosis is the result of any combination of fat substances, cholesterol, waste products, calcium as well as other substances being deposited on the inside lining of the artery. This buildup is often called plaque. Atherosclerosis can often lead to coronary heart disease, a major health concern today for both males and females in the United States as well as abroad.

Atherosclerosis may also lead to strokes, and other disorders because of the occurrence of blood clots, which may form in the narrowed arteries. Although plaques can grow large enough to significantly reduce the blood flow through an artery, most of the damage may occur when these plaques become fragile and rupture. These are often referred to as vulnerable plaques. When vulnerable plaques rupture they typically cause blood clots to form that may then subsequently block blood flow or break off and travel through the blood vessel to another part of the body. If either situation happens, the result may be a blocked blood vessel that supports and nourishes the heart, which may cause a heart attack. If a blood vessel that delivers blood to the brain is blocked, it may cause a stroke. If the blood supply to the legs is compromised, it may result in limb ischemia and in difficulty with walking and/or leg pain referred to as claudication, and may eventually cause gangrene.

The narrowing of an artery, also known as a stenotic lesion in the vasculature, has motivated medical professionals to develop a number of intravascular procedures which have evolved over time to treat this condition, percutaneous balloon angioplasty being the most common. Percutaneous balloon angioplasty is a procedure wherein a balloon catheter is inserted within the vasculature, and the balloon is expanded at the location of the lesion essentially compressing the stenotic buildup against the inside of the vessel wall. More recently this procedure has been augmented by the deployment of a stent or stents, at the location of the lesion subsequent to, or concurrently with the angioplasty. The stent acts as an internal scaffold within the vessel, retaining an open lumen and preventing further re-narrowing of the vessel. Generally, stents are primarily of two types, balloon expanding and self-expanding. As the terms indicate, balloon-expanding stents are deployed/expanded *in-vivo* with the assistance of a balloon, while self-expanding stents may utilize shape-memory materials such as nitinol. The use of such alloys as nitinol (Nickel-Titanium alloy), having shape memory characteristics, suitable biocompatibility, and designed to be inserted into one's vasculature, is known in the art. These so-called shape memory alloys have the ability to return to their original shape when exposed to the correct temperature conditions. The shape memory characteristics of these alloys, allow the devices to be deformed to facilitate their insertion into a body lumen or cavity. Upon being exposed to higher temperatures within the body results in the device returning to its original programmed shape. Thus one can employ the shape memory property to expand the device to its original shape after being delivered through the vasculature in a reduced profile or compressed state. Nitinol can also have super-elastic characteristics, which allow the device fabricated from such a material as nitinol, to be deformed and restrained in the deformed condition in order to facilitate the insertion into a patient's vasculature. This deformation causes a phase transformation of the material. Once the device with super-elastic characteristics is delivered, the restraint on the super-elastic material can be removed thus reducing the stress and allowing the previously compressed super-elastic member to return to its original pre-programmed and un-deformed shape, which results in a transformation back to the original phase.

While widespread intravascular procedures, particularly angioplasty procedures have been extremely successful; the procedure itself may result in development of an embolus. For example, during stent deployment and positioning, abrasion of the vessel wall may dislodge material resulting in an embolus. An embolus circulating within the blood vessels may lead to occlusion of a vessel and/or formation of clots within the vasculature and/or body organs. Although the occurrence of this can be minimized with careful and proper technique, when such an event does happen it may have serious consequences to the patient. By adequately capturing and/or filtering the material traveling in the blood responsible for causing such an event one can avoid the serious consequences that may result without such safeguards.

Multiple approaches to address capturing and/or filtering of the embolic debris/material from blood have been attempted. These include baskets, nets, suction, and even chemical modification of the debris (see US-5053008, which utilizes and requires an additional conduit to transport lysing agents to the trapped embolus). Use of vascular filters in the Vena Cava for capturing emboli has been disclosed (see US-4727873 and US-4688553). The designs of the Vena Cava filters continue to improve addressing such issues as fit and preventing migration (see US-6443972). Distal Protection devices using filtering baskets although similar to Vena Cava filters in function are typically temporarily positioned within the lumen, whereas Vena Cava filters are typically implanted within the vessel, most often the inferior Vena Cava, as the name implies. The use of a filtering basket positioned downstream from the procedure to capture the debris/material during an intravascular procedure is one such method. Because these basket type devices must be introduced into the vasculature and travel within the vasculature to be ultimately positioned to a location somewhat distal or downstream to the region of interest, most, if not all, incorporate and utilize concepts and features that make the essential delivery through the vessel less traumatic (see US-6391044). This can be accomplished by using a reduced size or compressed version of the apparatus. This allows one to deploy the apparatus to its normal working size when the apparatus is at the location of interest within the vessel.

The majority of these basket-type devices employ "expandable filters," meaning they may be fabricated from shape-memory materials which have the property that when exposed to the relatively elevated temperature within the body, they return to their initial programmed size. Alternately, on can rely on the superelastic property by removing the restraint on the geometry. These devices are generally placed distal, or downstream of the stenosis in order to capture any fragments, debris, and/or embolic material, that may be dislodged or occur as a result of the presence and use of the device during an intravascular procedure. The downstream placement of the device takes advantage of the blood flow within the vasculature, which will transport the undesirable material with it. The filtering membrane of the device is typically designed so as to allow blood flow through the membrane while limiting passage of the larger sized fragments and debris such as micro and macro emboli. These fragments, debris, and/or embolic material could potentially be carried beyond the device location with the blood flow downstream if not for such a filtering device as described herein. While this method performs fairly well capturing a substantial portion of the items intended to be captured; many of these designs are optimized for circular vessels. While outer vessel shapes are circular or slightly elliptical, the internal geometry of a vessel may often be non-circular as in an oval or elliptical shape or may even take the form of other non-circular geometries. Specifically, when calcification is present within the vessel, the internal vessel geometry is often irregular. Furthermore, vessel cross-sectional shape may vary with the type and location of vessel and vary across patients as well. Moreover, due to the circulation of blood through the vessel and resulting forces, a dynamic situation exists, which may produce additional geometry changes to the normal vessel shape. Thus expandable filters, which are generally designed for circular vessels, may result in a lack of apposition against the vessel wall over at least some portion of the internal circumference of the vessel wall when one takes into account the additional factors described above. Such a gap or leak path may occur when the resulting geometry of the expanded device is circular while the inner luminal cross-sectional shape of the vessel is more often non-circular. Such resulting gaps, between the device and inner luminal surface, may allow the emboli that the device is designed to capture, adequate room to pass through such a gap between the inner wall of the vessel and the outer confines of the device. When this occurs, the primary purpose of the device, which in this case is to capture fragments, debris and/or embolic material, is defeated, because the unfiltered flow path will allow for passage of the emboli. Even when the vessel itself is circular, conformance of a circular filter to the internal lumen of the vessel may not be optimal if "in-folding" is present. "In-folding" is the situation when the unsupported membrane of the system folds in at positions located between the strut locations where the membrane is supported by the struts. This situation can produce gaps between the inner vessel wall and the membrane even in the idealized circular vessel at locations between adjacent struts. In-folding may also occur when an oversized device (one that is sized larger then the vessel it will be placed in) is utilized in order to ensure adequate vessel coverage. In this situation, when the struts of the oversized device make contact with the vessel wall, the device ceases to expand, As a result, this limited expansion is short of its fully expanded programmed size and as such the membrane is not fully taught. Thus the remaining slack present in the membrane may lead to in-folding and thus allow for an unfiltered flow path. Moreover, even in the absence of in-folding, utilization of a circular-type device in a truly circular vessel may not adequately conform to the internal lumen upon vessel loading and/or deformation because the resulting device deformation may not adequately match the deformation of the vessel. This dissimilar deformation may thereby result in gaps or leak paths between the inner vessel wall and the device upon loading and/or deformation of the vessel.

International patent application publication no. WO-02/43595 discloses an embolic protection device for use in a blood vessel to capture embolic material which may be created and released into the bloodstream during an interventional procedure. The device includes a filtering assembly having a self-expanding strut assembly attached to the distal end of a guide wire deployed within the patient vasculature- A restraining sheath is placed over the filtering assembly in a coaxial arrangement to maintain the filtering assembly in its collapsed position until it is ready to be deployed. The sheath can then be retracted to expose the filtering assembly which then expands. The filtering element may be provided over the strut assembly.

International patent application publication no, WO-02/060519 discloses an embolic filtration system having a guide wire with a filter element attached thereto. The arrangement is such that the guide wire is free to rotate or translate while the filter element remains stationary. The system allows for movement and rotation of the guide wire as devices are advanced over it to treat occlusive disease, substantially without dislodging the filter element.

Accordingly, there is a need for a distal protection device with improved filtering and vessel apposition that can allow for capturing of embolic debris regardless of vessel size or shape or various loading regimes encountered by the vessel.

The distal protection device with improved apposition in accordance with the present invention overcomes the disadvantages and shortcomings of currently available devices and satisfies the unmet needs of maximizing capture of embolic debris by improved vessel apposition in vessels of varying size and shape in various loading and no-load regimes.

The present invention relates to an apparatus for intravascular filtering, capable of capturing emboli in blood flowing within the vasculature and a method of using the device. The filtering device comprises an expandable basket and is configured to deploy radially outward which may be relative to a centrally located guide wire. Expansion of the filtering device with improved vessel conformance is accomplished in a fashion resulting in improved filtering of blood in both circular and non-circular vessels and for vessels both large and small as well as when the vessel itself encounters various internal and/or external loading regimes. Furthermore the incorporation or application of biological and/or pharmaceutical agents can provide additional benefits when used in combination with the present invention.

In accordance with one exemplary embodiment of the present invention, the filtering basket and supporting struts work together to achieve the stated objective of improved conformance and apposition to the internal vessel wall. In this exemplary embodiment of the present invention where the filtering basket and struts work together, expansion of the filtering basket is decoupled from the struts, and as such, expansion of the filter basket can be independent of the expansion of the supporting struts that are operatively connected to the filtering basket. Operatively connected in this instance in accordance with an exemplary embodiments of the present invention equates to the struts connected to the membrane such that radial strut movement controls the radial expansion of the membrane but allows for independent longitudinal or axial movement of strut relative to the membrane. For example, in accordance with the present invention one can operatively attach the strut to the membrane by allowing the strut to slide within loops fixed to the membrane thereby allowing the radial expansion of the membrane to occur independent of the axial translation of the individual struts. In this exemplary embodiment, the independent expansion of each of the supporting struts can independently act on the filtering basket enabling the basket to expand as well, This expansion of the filtering basket may be non-uniform, As a result, improved vessel wall apposition is achieved by the independent radial expansion of each of the struts acting upon the filtering portion of the device. This improved conformance to the inside surface of an vessel wall results in improved filtering capacity by capturing emboli and/or other material which may otherwise be allowed to circumvent a device which does not conform to the vessel wall as closely. This provides a significant patient benefit.

The incorporation or application of biologically active or pharmaceutically active compounds with the present invention is a further object of this invention and is an improvement to methods and/or devices which require the use of a conduit to deliver the agent to the desired location. Compounds such as those identified below may be applied as coatings on these devices and may be used to deliver therapeutic and pharmaceutical agents which may include: anti-proliferative/antimitotic agents including natural products such as vinca alkaloids (i.e. vinblastine, vincristine, and vinorelbine), paclitaxel, epidipodophyllotoxins (i.e. etoposide, teniposide), antibiotics (dactinomycin (actinomycin D) dauriorubicin, doxorubicin and idarubicin), anthracyclines, mitoxantrone, bleomycins, plicamycin (mithramycin) and mitomycin, enzymes (L-asparaginase which systemically metabolizes L-asparagine and deprives cells which do not have the capacity to synthesize their own asparagine); antiplatelet agents such as G(GP) 11_{b}/111ₐ inhibitors and vitronectin receptor antagonists; anti-proliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nirtosoureas (carmustine (BCNU) and analogs, streptozocin), trazenes - dacarbazinine (DTIC); anti-proliferative/antimitotic antimetabolites such as folic acid analogs (methotrexate), pyrimidine analogs (fluorouracil, floxuridine, and cytarabine), purine analogs and related inhibitors (mercaptopurine, thioguanine, pentostatin and 2-chlorodeoxyadenosine {cladribine}); platinum coordination complexes (cisplatin, carboplatin), procarbazine, hydroxyurea, mitotane, aminoglutethimide; hormones (i.e. estrogen); anti-coagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory; antisecretory (breveldin); anti-inflammatory: such as adrenocortical steroids (cortisol, cortisone, fludrocortisone, prednisone, prednisolone, 6α-methylprednisolone, triamcinolone, betamethasone, and dexamethasone), non-steroidal agents (salicylic acid derivatives i.e. aspirin; para-aminophenol derivatives i.e. acetaminophen; indole and indene acetic acids (indomethacin, sulindac, and etodalac), heteroaryl acetic acids (tolmetin, diclofenac, and ketorolac), arylpropionic acids (ibuprofen and derivatives), anthranilic acids (mefenamic acid, and meclofenamic acid), enolic acids (piroxicam, tenoxicam, phenylbutazone, and oxyphenthatrazone), nabumetone, gold compounds (auranofin, aurothioglucose, gold sodium thiomalate); immunosuppressives: (cyclosporine, tacrolimus (FK-506), sirolimus (rapamycin), azathioprine, mycophenolate mofetil); angiogenic agents: vascular endothelial growth factor (VEGF), fibroblast growth factor (FGF); angiotensin receptor blockers; nitric oxide donors; antisense oligionucleotides and combinations thereof; cell cycle inhibitors, mTOR inhibitors, and growth factor receptor signal transduction kinase inhibitors; retenoids; cyclin/CDK inhibitors; HMG co-enzyme reductase inhibitors (statins); and protease inhibitors.

The use of compounds in conjunction with the present invention can provide distinct clinical advantages over existing therapies and/or devices. More specifically, compounds that are capable of causing lysis or degradation of the embolic debris can be incorporated into the filtering portion of the present invention. A factor to consider in the selection of such a compound is the origin of the debris be it thrombus, plaque, atheroma, or any other form representing an embolus. As the mesh and or pore size of the filtering aspect of the present invention decreases, more embolic material may become trapped in the filtering mechanism of the present invention, thereby increasing the load on the filtering portion. While small emboli (typically smaller than 100 microns) are not a major concern because of the body's natural ability to enzymatically degrade, digest or lyse the emboli, the embolic load on the filter itself can be overloaded and result in formation of a thrombus if the blood flow is significantly slowed to the point which allows for a thrombus formation. In this situation the incorporation or application of compounds, which can degrade trapped emboli, can be beneficial. Some exemplary suitable compounds may include: Tissue Plasminogen(TPA); Streptokinase(SK); Reteplase; Tenecteplase; Urokinase; Lanoteplase; Staphylokinase; and/or Nadroparin(anti-factor Xa). In addition, the filtering portion of the present invention may incorporate an antithrombotic and/or antithrombogenic agent to prevent the formation of a thrombus. Some exemplary compounds may include: Heparin; Fragmin (dalteparin, low MW Heparin); a monoclonal antibody such as ReoPro™ (abciximab, antiplatelet antibodies) Acenocoumarol; Anisindione; Dicumarol; Warfarin; Enoxaparin (Lovenox); Anagrelide (Agrylin); Indomethacin (Indocin); Dipyridamole; Clopidogrel; Aggrenox; and/or Coumadin. Furthermore, an affinity-binding compound may also be incorporated with the filtering aspect of the present invention by itself or in combination with other compounds. Affinity-binding compounds can promote the binding and/or adhesion of embolic material thus facilitating entrapment of embolic material and subsequent removal from the blood stream. Whether incorporated into the strut or membrane by methods such as chemical surface treatments, bombardment, placement into reservoirs, or in the case of polymeric struts and membranes, blended with the material itself, or by application of a coating to the struts and/or membrane with a compound, any identified compound or combination of identified compounds may be used. Furthermore any number of compounds may suggest themselves to one who is skilled in the art and may be utilized in connection with the present invention alone or in combination with other compounds.

The foregoing exemplary embodiments of the present invention each provide a reliable, easy to manufacture, and simple to use device that significantly improves wall apposition of the outer confines of the device to the internal surface of the vessel wall regardless of vessel size or shape or loading regime encountered. Furthermore, a relatively reduced profile of the delivered device is achievable in accordance with the present invention. Moreover, any combination of the items identified that are capable of expansion and provide for the ability to independently conform to both circular and non-circular geometries upon expansion may be utilized. As noted above, the incorporation of biological and/or pharmaceutically active agents with the present invention can be utilized for the additional purposes of preventing thrombus formation, promotion of binding, and degradation of thrombus, all of which provide a patient benefit.

Embodiments of the invention will now be described by way of example with reference to the accompanying drawings, in which:
Figures 1a & 1b are planar views showing the disparate cross-sectional coverage area of an existing prior art filtering device in both a circular vessel and a non-circular vessel and the resulting gap in coverage due to non-conformance of the device when positioned in non-circular vessels;
Figures 2a & 2b are planar views showing the disparate cross-sectional coverage area by a filter with improved vessel conformance in accordance with the present invention in both a circular and non-circular vessel. The schematic in figure 2b shows the minimization of gaps particularly in a non-circular vessel that results in contrast to the result achieved with prior art devices shown schematically in figure 1b; and
Figure 3 is a partial side view of the filtering device in accordance with the present invention positioned on a guide wire.

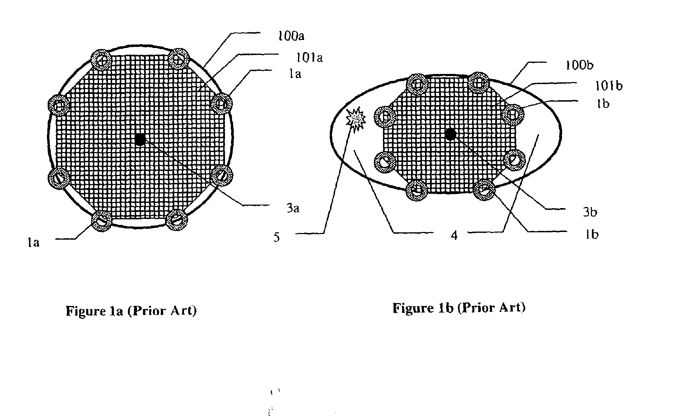

Figures 1a & 1b show a cross-sectional view for both a circular (100a) and a non-circular (100b) blood vessel. Superimposed within these vessels in figures 1a & 1b are schematics showing the approximated or relative cross-sectional coverage area (101a) and (101 b) for typical existing prior art devices in both circular (100a) and non-circular (100b) idealized vessels. In this case the vessel wall (100a) & (100b) constrains the extent of the expansion of devices located within the circumference of the vessel. With existing devices that uniformly expand, this expansion is such that strut point locations represented by (1a) & (1 b) in the circular and non-circular vessels respectively, are uniformly equidistant from the central axis (3a & 3b). As such, when strut point locations (1b) come into contact with the vessel wall (100b), at the moment one or more struts make contact, all subsequent expansion is halted. As shown, given that all strut point locations (1b) expand in unison and uniformly, the right two and left two strut point locations (1b) never make contact with the internal vessel wall surface (100b) because the top two and bottom two strut point locations (1b) make prior contact with the vessel wall (100b) and thus the entire device is prevented from further expansion. This results in a gap of coverage area (4) that may allow embolic material (5) to flow past the filtering device. The gap in coverage area is not only present in non-circular vessels, but depending on the extent of non-circularity of the vessel may result in further increasing the gap present and may also occur or increase due to various vessel loading situations.

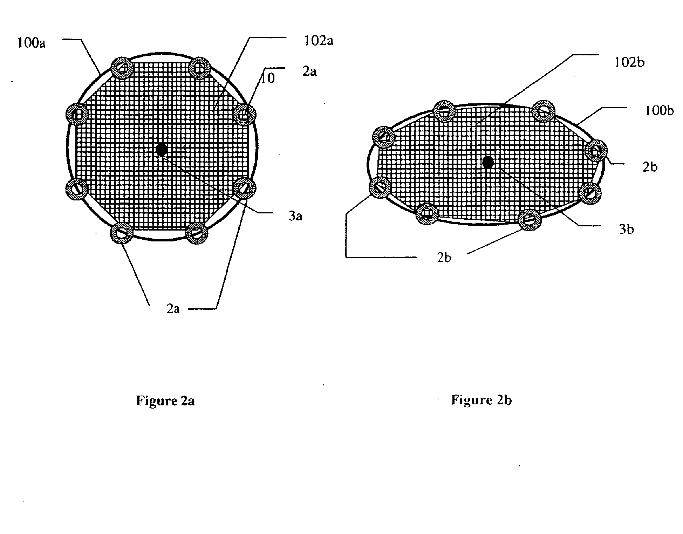

This is in contrast with the result achieved with an improved vessel conformance device in accordance with the present invention. A similar set of schematics represented in Figures 2a & 2b show the cross-sectional coverage area and obtainable results in accordance with the present invention in both circular (100a) and non-circular (100b) vessels. Figures 2a & 2b show cross-sectional views for both a circular (100a) and a non-circular (100b) blood vessel. Superimposed within this vessel is a schematic showing the cross-sectional coverage area (102a) and (102b) for an improved vessel conformance devices in accordance with the present invention in both circular (100a) and non-circular (100b) vessels. In this case the vessel wall (100a) & (100b) constrains the extent of the expansion of devices located within the circumference of the vessel as before. However because the strut point locations (2a & 2b) are decoupled from the filtering portion these strut points can continue to expand independently of each other and the filtering basket, even when one strut point location makes contact with the vessel wall. Thus the outward expansion of the other strut points (2b) are not inhibited and thus can continue to expand until each independently makes contact with the vessel wall regardless of the nature of the cross-sectional shape of the vessel. This results in minimization and/or avoidance of any gap in coverage area.

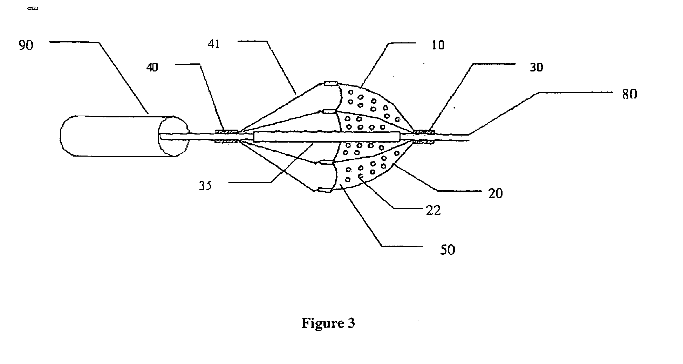

Figure 3 shows a side view of an exemplary embodiment of the present invention, positioned on a guide wire (80). The filter basket (10) includes one or more struts (20), which serve to provide support and impart shape to the filter membrane (50). The filter membrane (50) in this exemplary embodiment is shown with uniform holes (22), but can also have a distribution of varying diameter holes to improve filtering performance or optimize filtering performance to achieve the desired result. Any number of designs may suggest themselves to one who is a skilled artisan in the field and may be utilized in connection with the present invention. The distal ends of the struts (20) are pivotally attached to supporting collar (30), which may be permanently, removably, or operatively attached to guide wire (80). When operatively attached, supporting collar (30) can slide and/or rotate with respect to guide wire (80). The struts (20) are operatively attached to the filter membrane (50) which taken together form the filter basket (10). The distal ends of additional tension members (41), attach to proximal ends of struts (20), while the proximal ends of these tension members (41) are fixed to the closure ring (40). When proximal movement (ie: away from supporting collar (30)) is imparted to closure ring (40) this increases the tension in tension members (41) such that strut members (20) are pulled radially inward toward guide wire (80) allowing for filter basket (10) comprised of filter membrane (50) and struts (20) to be collapsed with any retained debris to be captured in basket. The entire assembly can then be pulled back into retaining sheath (90). An inner sleeve (35) whose axial position relative to guide wire (80) can impart motion to either closure ring (40) or supporting collar (30) in order to draw the assembly into the retaining sheath (90) or to deliver the assembly from the retaining sheath (90) may also be present. The pore size of filter membrane (50) can be optimized by altering the diameters of the individual pores (22) as well as their location of different sized pores relative to each other in order to maximize optimal blood flow through the membrane (50) while still filtering debris for whose size is of consequence. Preferred pore size diameters of individual pores (22) of the present invention range from approximately 50 to 150 µm (50 to 150 microns), however any number of suitable combinations of large and small size pores is also possible as is the distribution of said pores with respect to the filter membrane (50).

In each exemplary embodiment, the device including both struts (20) and proximal support ring (40) may be cut from a single tube eliminating the need for separate structural components. As an example, laser cutting techniques for stent manufacturing can be employed to fabricate the embodiments described in accordance with the present invention. Cutting all or most of the structural components from a single tube by laser cutting or other appropriate methods provides significant cost savings as a result of the reduced number of manufacturing process steps. In certain instances, formed wire may also be used to fabricate the device in accordance with the present invention. Some device designs and shapes simply do not lend themselves to cost effective laser cutting and thus wire forming would be more cost effective. Supporting struts (20) can be fabricated from a number of biocompatible materials including metals, ceramics, and polymers. Preferable materials for the supporting struts (20) are shape memory metals and super-elastic alloys such as nitinol.

## Claims

1. A vascular filtering device comprising:
a supporting collar (30) having a concentric through hole adapted to slidably engage with a guide wire (80);
a primary supporting strut (20) having a proximal end and a distal end wherein the distal end of said supporting strut (20) is operatively attached to said supporting collar (30) and the proximal end of said supporting strut (20) is free to expand away from a guide wire (80); and
a compliant substantially conical membrane (50), **characterised in that** said membrane (50) is operatively attached to both said supporting strut (20) and to said supporting collar (30), such that radial movement of the supporting strut (20) away from a guide wire (80) causes radial movement of the membrane (50) but allows for independent longitudinal or axial movement of the supporting strut (20) relative to the membrane (50).

2. The filtering device of claim 1 further comprising one or more additional supporting struts (20) each having a proximal end and a distal end, spaced apart from said primary supporting strut (20) wherein said membrane (50) is operatively attached to each of said additional supporting struts (20) and wherein the distal end of each of said additional supporting struts (20) is operatively attached to said supporting collar (30) and the proximal end of each of said additional supporting struts (20) is free to expand away from the guide wire (80),

3. The filtering device of claim 1 or claim 2 wherein the membrane (50) is operatively attached to the or each supporting strut (20) by allowing the or each strut (20) to slide within loops fixed to the membrane (50).

4. The filtering device of claim 2 further comprising:
a closure ring (40) having a concentric though hole adapted to slidably engage with the guide wire (80) positioned proximally to said supporting collar (30); and
a tension member (41) having a proximal end and a distal end wherein the proximal end of said tension member (41) is attached to said closure ring (40) and the distal end of said tension member (41) is attached to the proximal end of said primary supporting strut (20).

5. The filtering device of claim 4 further comprising an inner sleeve (35) positioned proximal to said supporting collar (30) and distal to said closure ring (40), fixable to the guidewire (80).

6. The filtering device of claim 4 wherein said closure ring (40) is fixable to the guidewire (80) and further comprises an inner stop positioned proximal to said supporting collar (30) and distal to said closure ring (40).

7. The filtering device of claim 1 wherein said membrane (50) is a polymeric film.

8. The filtering device of claim 7 wherein said polymeric film is a polyurethane having at least one through hole (22).

9. The filtering device as in claim 1 wherein said membrane (50) is a Nickel-Titanium Alloy thin film having at least one through hole (22).

10. The filtering device of claim 1 wherein said strut (20) is metal.

11. The filtering device or claim 2 wherein said struts (20) are metal.

12. The filtering device of claim 1 wherein said membrane (50) incorporates an active compound.

13. The filtering device of claim 1 wherein said strut (20) incorporates an active compound.

14. The filtering device of claim 2 wherein said struts (20) incorporate an active compound.

15. The filtering device of claim 1 wherein said strut (20) is polymeric.

16. The filtering device of claim 1 wherein said strut (20) is ceramic.

17. The filtering device of claim 10 or 11 wherein said metal is a shape-memory alloy.

18. The filtering device of claim 10 or 11 wherein said metal is a super-elastic alloy.

19. The filtering device of claim 17 wherein said shape-memory alloy is Nickel-Titanium Alloy.

20. The filtering device of claim 18 wherein said super-elastic alloy is Nickel-Titanium Alloy.

21. A method for fabricating a filtering device according to claim 1 comprising the steps of:
a. providing a hollow tube; and
b. removing material from said tube such that what remains are a distal support collar (3 0), having a concentric through hole adapted to slidably engage with a guide wire (80) and strut members (20) each of which are integrally attached to each other being formed from the same tubular component;
wherein a free end of each strut member (20) is free to expand away from a guide wire (80), the method further comprising providing a compliant substantially conical membrane (50), wherein said membrane (50) is operatively attached to both said strut members (20) and to said support collar (30), such that radial movement of the support members (20) away from a guide wire (80) causes radial movement of the membrane (50) but allows for independent longitudinal or axial movement of the support members (20) relative to the membrane (50).

22. The method of claim 21 wherein said tube is Nickel-Titanium Alloy.

23. The method of claim 21 wherein said removal of material is performed by laser cutting,

24. A method for fabricating a filtering device acccording to claim 1 comprising the steps of:
c. providing elongated wire;
d. forming said elongated wire into strut members (20); and
e. attaching said strut members (20) to a support collar (30) having a concentric through hole adapted to slidably engage with a guide wire (80);
wherein a free end of each strut member (20) is free to expand away from a guide wire (80), the method further comprising providing a compliant substantially conical membrane (50), wherein said membrane (50) is operatively attached to both said strut members (20) and to said support collar (30), such that radial movement of the support members (20) away from a guide wire (80) causes radial movement of the membrane (50) but allows for independent longitudinal or axial movement of the support members (20) relative to the membrane (50).

25. The method of claim 24 wherein said elongated wire is Nickel-Titanium Alloy.

## Patentansprüche

1. Vaskuläre Filtervorrichtung, welche Folgendes umfasst:
einen Stützkragen (30), welcher ein konzentrisches Durchgangsloch aufweist, welches geeignet ist, um verschiebbar mit einem Führungsdraht (80) einzukoppeln; eine primäre Stützstrebe (20), welche ein proximales Ende und ein distales Ende aufweist, wobei das distale Ende der Stützstrebe (20) wirksam an dem Stützkragen (30) befestigt ist und das proximale Ende der Stützstrebe (20) frei ist, um von einem Führungsdraht (80) weg zu expandieren; und
eine nachgiebige, im Wesentlichen konische Membran (50), **dadurch gekennzeichnet, dass** die Membran (50) wirksam sowohl an der Stützstrebe (20) als auch an dem Stützkragen (30) befestigt ist, sodass eine radiale Bewegung der Stützstrebe (20) von einem Führungsdraht (80) weg eine radiale Bewegung der Membran (50) bewirkt, aber eine unabhängige longitudinale oder axiale Bewegung der Stützstrebe (20) relativ zu der Membran (50) ermöglicht ist.

2. Filtervorrichtung nach Anspruch 1, welche weiterhin eine oder mehrere zusätzliche Stützstreben (20) umfasst, wobei jede ein proximales Ende und ein distales Ende aufweist und von der primären Stützstrebe (20) beabstandet ist, wobei die Membran (50) wirksam an jeder der zusätzlichen Stützstreben (20) befestigt ist, und wobei das distale Ende jeder der zusätzlichen Stützstreben (20) wirksam an dem Stützkragen (30) befestigt ist und das proximale Ende jeder der zusätzlichen Stützstreben (20) frei ist, um von dem Führungsdraht (80) weg zu expandieren.

3. Filtervorrichtung nach Anspruch 1 oder Anspruch 2, wobei die Membran (50) wirksam an der oder jeder Stützstrebe (20) befestigt ist, indem der oder jeder Strebe (20) ermöglicht ist, innerhalb von Schlaufen, welche an der Membran (50) befestigt sind, verschoben zu werden.

4. Filtervorrichtung nach Anspruch 2, welche weiterhin Folgendes umfasst:
einen Schlussring (40), welcher ein konzentrisches Durchgangsloch aufweist, welches geeignet ist, um verschiebbar mit dem Führungsdraht (80) einzukoppeln, welcher proximal zu dem Stützkragen (30) positioniert ist; und
ein Zugglied (41), welches ein proximales Ende und ein distales Ende aufweist,
wobei das proximale Ende des Zuggliedes (41) an dem Schlussring (40) befestigt ist und das distale Ende des Zuggliedes (41) an dem proximalen Ende der primären Stützstrebe (20) befestigt ist.

5. Filtervorrichtung nach Anspruch 4, welche weiterhin eine innere Hülse (35) umfasst, welche proximal zu dem Stützkragen (30) und distal zu dem Schlussring (40) positioniert ist und an dem Führungsdraht (80) fixierbar ist.

6. Filtervorrichtung nach Anspruch 4, wobei der Schlussring (40) an dem Führungsdraht (80) fixierbar ist und weiterhin einen inneren Anschlag umfasst, welcher proximal zu dem Stützkragen (30) und distal zu dem Schlussring (40) positioniert ist.

7. Filtervorrichtung nach Anspruch 1, wobei die Membran (50) ein Polymerfilm ist.

8. Filtervorrichtung nach Anspruch 7, wobei der Polymerfilm ein Polyurethan ist, welches zumindest ein Durchgangsloch (22) aufweist.

9. Filtervorrichtung wie in Anspruch 1, wobei die Membran (50) ein dünner Nickel-Titan-Legierungsfilm ist, welcher zumindest ein Durchgangsloch (22) aufweist.

10. Filtervorrichtung nach Anspruch 1, wobei die Strebe (20) metallen ist.

11. Filtervorrichtung nach Anspruch 2, wobei die Streben (20) metallen sind.

12. Filtervorrichtung nach Anspruch 1, wobei die Membran (50) eine aktive Verbindung aufweist.

13. Filtervorrichtung nach Anspruch 1, wobei die Strebe (20) eine aktive Verbindung aufweist.

14. Filtervorrichtung nach Anspruch 2, wobei die Streben (20) eine aktive Verbindung aufweisen.

15. Filtervorrichtung nach Anspruch 1, wobei die Strebe (20) polymer ist.

16. Filtervorrichtung nach Anspruch 1, wobei die Strebe (20) keramisch ist.

17. Filtervorrichtung nach Anspruch 10 oder 11, wobei das Metall eine Formgedächtnislegierung ist.

18. Filtervorrichtung nach Anspruch 10 oder 11, wobei das Metall eine superelastische Legierung ist.

19. Filtervorrichtung nach Anspruch 17, wobei die Formgedächtnislegierung eine Nickel-Titan-Legierung ist.

20. Filtervorrichtung nach Anspruch 18, wobei die superelastische Legierung eine Nickel-Titan-Legierung ist.

21. Verfahren zum Herstellen einer Filtervorrichtung nach Anspruch 1, welches die folgenden Schritte umfasst:
a) Bereitstellen einer hohlen Röhre; und
b) Entfernen von Material aus der Röhre, sodass jenes, welches übrig bleibt, ein distaler Stützkragen (30), welcher ein konzentrisches Durchgangsloch aufweist, welches geeignet ist, um verschiebbar mit einem Führungsdraht (80) einzukoppeln, und Strebenelemente (20) sind, wobei jedes davon integral an jedem anderen, welches aus der gleichen röhrenförmigen Komponente gebildet ist, befestigt ist;
wobei ein freies Ende jedes Strebenelements (20) frei ist, um von einem Führungsdraht (80) weg zu expandieren, wobei das Verfahren weiterhin ein Bereitstellen einer nachgiebigen, im Wesentlichen konischen Membran (50) umfasst,
wobei die Membran (50) wirksam sowohl an den Strebenelementen (20) als auch an dem Stützkragen (30) befestigt ist, sodass eine radiale Bewegung der Stützelemente (20) von einem Führungsdraht (80) weg eine radiale Bewegung der Membran (50) bewirkt, aber eine unabhängige longitudinale oder axiale Bewegung der Stützelemente (20) relativ zu der Membran (50) ermöglicht ist.

22. Verfahren nach Anspruch 21, wobei die Röhre eine Nickel-Titan-Legierung ist.

23. Verfahren nach Anspruch 21, wobei das Entfernen von Material durch ein Laserschneiden durchgeführt wird.

24. Verfahren zum Herstellen einer Filtervorrichtung nach Anspruch 1, welches folgende Schritte umfasst:
c) Bereitstellen eines länglichen Drahtes;
d) Umformen des länglichen Drahtes in Strebenelemente (20); und
e) Befestigen der Strebenelemente (20) an einem Stützkragen (30), welcher ein konzentrisches Durchgangsloch aufweist, welches geeignet ist, um verschiebbar mit einem Führungsdraht (80) einzukoppeln;
wobei ein freies Ende jedes Strebenelements (20) frei ist, um von einem Führungsdraht (80) weg zu expandieren, wobei das Verfahren weiterhin ein Bereitstellen einer nachgiebigen, im Wesentlichen konischen Membran (50) umfasst, wobei die Membran (50) wirksam sowohl an den Strebenelementen (20) als auch an dem Stützkragen (30) befestigt ist, sodass eine radiale Bewegung der Stützelemente (20) von einem Führungsdraht (80) weg eine radiale Bewegung der Membran (50) bewirkt, aber eine unabhängige longitudinale oder axiale Bewegung der Stützelemente (20) relativ zu der Membran (50) ermöglicht ist.

25. Verfahren nach Anspruch 24, wobei der längliche Draht eine Nickel-Titan-Legierung ist.

## Revendications

1. Dispositif de filtrage vasculaire comprenant :
> un collier de support (30) qui présente un trou traversant concentrique adapté de manière à venir en prise de façon coulissante avec un fil guide (80) ;
> une entretoise de support (20) primaire qui présente une extrémité proximale et une extrémité distale dans laquelle l'extrémité distale de ladite entretoise de support (20) est fixée de manière opérationnelle audit collier de support (30) et l'extrémité proximale de ladite entretoise de support (20) est libre de s'étendre en allant en s'éloignant d'un fil guide (80) ; et
> une membrane (50) sensiblement conique et conforme, **caractérisé en ce que** ladite membrane (50) est fixée de manière opérationnelle sur ladite entretoise de support (20) et sur ledit collier de support (30), de telle sorte qu'un déplacement radial de l'entretoise de support (20) en allant s'éloignant du fil guide (80) provoque un déplacement radial de la membrane (50) mais permette un déplacement longitudinal ou axial indépendant de l'entretoise de support (20) par rapport à la membrane (50).

2. Dispositif de filtrage selon la revendication 1, comprenant en outre une ou plusieurs entretoises de support (20) supplémentaires, chacune d'elles présentant une extrémité proximale et une extrémité distale, espacées de ladite entretoise de support (20) primaire, dans lequel ladite membrane (50) est fixée de manière opérationnelle à chacune desdites entretoises de support (20) supplémentaires et dans lequel l'extrémité distale de chacune desdites entretoises de support (20) supplémentaires est fixée de manière opérationnelle audit collier de support (30) et l'extrémité proximale de chacune desdites entretoises de support (20) supplémentaires est libre de s'étendre en allant en s'éloignant du fil guide (80).

3. Dispositif de filtrage selon la revendication 1 ou la revendication 2, dans lequel la membrane (50) est fixée de manière opérationnelle à chacune des entretoises de support (20) en permettant à chacune des entretoises (20) de coulisser à l'intérieur de boucles fixées à la membrane (50).

4. Dispositif de filtrage selon la revendication 2, comprenant en outre:
> un anneau de fermeture (40) qui présente un trou traversant concentrique adapté de manière à venir en prise de façon coulissante avec le fil guide (80) placé de façon proximale par rapport audit collier de support (30) ; et
> un élément de tension (41) qui présente une extrémité proximale et une extrémité distale dans lequel l'extrémité proximale dudit élément de tension (41) est fixée audit anneau de fermeture (40) et l'extrémité distale dudit élément de tension (41) est fixée à l'extrémité proximale de ladite entretoise de support (20) primaire.

5. Dispositif de filtrage selon la revendication 4, comprenant en outre un manchon intérieur (35) placé proximal par rapport audit collier de support (30) et distal par rapport audit anneau de fermeture (40), et pouvant être fixé sur le fil guide (80).

6. Dispositif de filtrage selon la revendication 4, dans lequel ledit anneau de fermeture (40) peut être fixé sur le fil guide (80) et comprend en outre un arrêt intérieur placé proximal par rapport audit collier de support (30) et distal par rapport audit anneau de fermeture (40).

7. Dispositif de filtrage selon la revendication 1, dans lequel ladite membrane est constituée d'une couche polymère.

8. Dispositif de filtrage selon la revendication 7, dans lequel ladite couche polymère est en polyuréthane et présente au moins un trou traversant (22).

9. Dispositif de filtrage selon la revendication 1, dans lequel ladite membrane (50) est constituée d'une couche mince d'alliage de nickel - titane qui présente au moins un trou traversant (22).

10. Dispositif de filtrage selon la revendication 1, dans lequel ladite entretoise (20) est en métal.

11. Dispositif de filtrage selon la revendication 2, dans lequel lesdites entretoises (20) sont en métal.

12. Dispositif de filtrage selon la revendication 1, dans lequel ladite membrane (50) incorpore un composé actif.

13. Dispositif de filtrage selon la revendication 1, dans lequel ladite entretoise (20) incorpore un composé actif.

14. Dispositif de filtrage selon la revendication 2, dans lequel lesdites entretoises (20) incorporent un composé actif.

15. Dispositif de filtrage selon la revendication 1, dans lequel ladite entretoise (20) est constituée d'un polymère.

16. Dispositif de filtrage selon la revendication 1, dans lequel ladite entretoise (20) est constituée d'une céramique.

17. Dispositif de filtrage selon la revendication 10 ou la revendication 11, dans lequel ledit métal est un alliage à mémoire de forme.

18. Dispositif de filtrage selon la revendication 10 ou la revendication 11, dans lequel ledit métal est un alliage super-élastique.

19. Dispositif de filtrage selon la revendication 17, dans lequel ledit alliage à mémoire de forme est un alliage de nickel - titane.

20. Dispositif de filtrage selon la revendication 18, dans lequel ledit alliage super-élastique est un alliage de nickel - titane.

21. Procédé de fabrication d'un dispositif de filtrage selon la revendication 1, comprenant les étapes consistant à :
a. fournir un tube creux ; et
b. retirer du matériau dudit tube de telle sorte qu'il reste un collier de support distal (30) qui présente un trou traversant concentrique adapté de manière à venir en prise de façon coulissante avec un fil guide (80) et des éléments d'entretoises (20), chacun d'eux étant fixé d'une pièce les uns aux autres en étant formés à partir du même composant tubulaire ;
dans lequel une extrémité libre de chaque élément d'entretoise (20) est libre de s'étendre en allant en s'éloignant d'un fil guide (80), le procédé comprenant en outre l'étape consistant à fournir une membrane sensiblement conique conforme (50), dans lequel ladite membrane (50) est fixée de manière opérationnelle auxdits éléments d'entretoises (20) et audit collier de support (30), de telle sorte qu'un déplacement radial des éléments de support (20) en allant en s'éloignant d'un fil guide (80) provoque un déplacement radial de la membrane (50) mais permette un déplacement longitudinal ou axial indépendant des éléments de support (20) par rapport à la membrane (50).

22. Procédé selon la revendication 21, dans lequel ledit tube est constitué d'un alliage de nickel - titane.

23. Procédé selon la revendication 21, dans lequel ledit retrait de matériau est exécuté par usinage par laser.

24. Procédé de fabrication d'un dispositif de filtrage selon la revendication 1, comprenant les étapes consistant à :
c. fournir un fil allongé ;
d. façonner ledit fil allongé en éléments d'entretoises (20) ; et
e. fixer lesdits éléments d'entretoises (20) sur un collier de support (30) qui présente un trou traversant concentrique adapté de manière à venir en prise de façon coulissante avec un fil guide (80) ;
dans lequel une extrémité libre de chaque élément d'entretoise (20) est libre de s'étendre en allant en s'éloignant d'un fil guide (80), le procédé comprenant en outre l'étape consistant à fournir une membrane sensiblement conique conforme (50), dans lequel ladite membrane (50) est fixée de manière opérationnelle auxdits éléments d'entretoises (20) et audit collier de support (30), de telle sorte qu'un déplacement radial des éléments de support (20) en allant en s'éloignant d'un fil guide (80) provoque un déplacement radial de la membrane (50) mais permette un déplacement longitudinal ou axial indépendant des éléments de support (20) par rapport à la membrane (50).

25. Procédé selon la revendication 24, dans lequel ledit fil allongé est constitué d'un alliage de nickel - titane.
